# EUROPEAN PATENT APPLICATION

(11) **EP 1 637 599 A2**
(43) Date of publication of application: **22.03.2006**
(21) Application number: 05017881.3
(22) Date of filing: 17.08.2005
(51) Int. Cl.: C12N 15/10

(54) **Method of nucleic acid isolation**

(30) Priority: 17.09.2004 JP 2004270657
(71) Applicant: Hitachi High-Technologies Corporation, Tokyo 105-8717 (JP)
(72) Inventor: Yamashita, Yoshihiro, 882 Ichige Hitachinaka Ibaraki 312-8504 (JP); Sakurai, Toshinari, 882 Ichige Hitachinaka Ibaraki 312-8504 (JP)
(74) Representative: Strehl Schübel-Hopf & Partner

(57) **Abstract**

It is an object of the present invention to release and isolate nucleic acid from a biological sample readily and in a short time. The present invention relates to a technique for isolating nucleic acid by which a biological sample including cells is caused to pass through a solid substance carrier to isolate the cells, a mixture of the cells and a cell lysis reagent is caused to pass through the solid substance carrier to disrupt the cells and release nucleic acid therein, and a mixture of the released nucleic acid and a nucleic acid binding reagent is caused to pass through the solid substance carrier to bind the nucleic acid to the solid substance carrier. Examples of an instrument comprising such solid substance carrier include an instrument comprising a syringe having a solid substance carrier fixed therein. The instrument is capable of moving a solution from one space to another space separated by the solid substance carrier, using pressurization and depressurization via the syringe, thereby causing the solution to pass through the solid substance carrier. Preferably, the solid substance carrier is a solid substance having a multitude of channels therein or a mesh-like solid substance composed of fibrous substances, by which cells are disrupted from a biological sample including the cells, nucleic acid is released, and the nucleic acid can be bound.

## Description

### BACKGROUND OF THE INVENTION

### Field of the Invention

The present invention relates to a technique for isolating nucleic acid from a biological sample. For example, the present invention relates to a technique for isolating RNA from leukocytes included in whole blood.

### Background Art

Gene information obtained by analysis of nucleic acid is utilized in various fields such as medical care, clinical examinations, pharmaceutical industry, food industry, and the like. Such analysis of nucleic acid requires a preprocessing operation consisting of isolating nucleic acid, where nucleic acid is isolated and purified from cells included in a biological sample such as blood, blood serum, urine, or the like. The nucleic acid must be isolated with a high degree of purification such that it does not contain any substances that would degrade the properties of nucleic acid or substances that would be factors preventing nucleic acid analysis.

Nucleic acid isolation methods generally include a method reported in Non-patent Document 1 that is based on the property of nucleic acid binding to silica in the presence of a chaotropic agent. Another method is based on the property of nucleic acid binding to silica in the presence of an organic solvent as disclosed in Patent Documents 1 and 2.

However, in order to isolate nucleic acid with a high degree of purification, nucleic acid isolation is preferably conducted after cells are isolated from a biological sample and then disrupted, thereby releasing nucleic acid.

For example, Non-patent Document 2 discloses a technique for isolating nucleic acid where RNA is extracted from isolated leukocytes after erythrocytes have been removed from blood. Specifically, after the erythrocytes in blood are dissolved using a red blood cell lysis reagent, leukocytes are isolated by centrifugation. Thereafter, a chaotropic salt solution having protein-denaturing effects and RNase-inactivating effects is added to the isolated leukocytes, and the leukocytes are dissolved using a homogenizer. Then ethanol is added to the leukocytes solution and a siliceous solid substance carrier is brought into contact with the leukocytes solution to cause RNA to bind to the solid substance carrier. After impurities are washed and removed form the solid substance carrier, RNA is eluted.
Patent Document 1: JP Patent Publication (Kokai) No. 2001-95572 A
Patent Document 2: JP Patent Publication (Kokai) No. 2002-360245 A
Non-patent Document 1: B. Vogelstein and D. Gillespie, Proc. Natl. Acad. Sci. U. S. A., 76 (2), 615-619 (1979)
Non-patent Document 2: QlAamp RNA Blood Mini, Protocol and trouble shooting, October, 2000

### SUMMARY OF THE INVENTION

The nucleic acid isolation method disclosed in Non-patent Document 2 enables nucleic acid to be obtained with a high degree of purification. However, it requires various devices and instruments, such as a centrifugal separator, a homogenizer, and instruments for isolating nucleic acid, so that the operation for isolating nucleic acid is complicated and requires a significant amount of time. As the time required for isolating nucleic acid increases, the problem of degradation of the properties of isolated nucleic acid becomes more evident.

It is therefore an object of the present invention to allow nucleic acid to be released and isolated from a biological sample readily and in a short time.

The present invention relates to a technique for isolating nucleic acid by which a biological sample including cells is caused to pass through a solid substance carrier to isolate the cells, a mixture of the cells and a cell lysis reagent is caused to pass through the solid substance carrier to disrupt the cells and release nucleic acid therein, and a mixture of the released nucleic acid and a nucleic acid binding reagent is caused to pass through the solid substance carrier to bind the nucleic acid to the solid substance carrier.

Also, the present invention relates to a technique for isolating nucleic acid by which a mixture of cells and a cell lysis reagent is caused to pass through a solid substance carrier to disrupt the cells and release nucleic acid therein, and a mixture of the released nucleic acid and a nucleic acid binding reagent is caused to pass through the solid substance carrier to bind the nucleic acid to the solid substance carrier.

Further, the present invention relates to a technique for releasing nucleic acid by which a biological sample including cells is caused to pass through a solid substance carrier to isolate the cells, and a mixture of the cells and a cell lysis reagent is caused to pass through the solid substance carrier to disrupt the cells and release nucleic acid therein.

Examples of instruments comprising a solid substance carrier include an instrument comprising a syringe having a solid substance carrier fixed therein, an instrument comprising a chip having a solid substance carrier fixed at the tip thereof, and an instrument comprising a spin column having a solid substance carrier fixed thereto that is adapted to be mounted on a centrifugal separator. The instrument comprising a syringe having a solid substance carrier fixed therein is capable of moving a solution from one space to another space separated by the solid substance carrier, using pressurization and depressurization via the syringe, thereby causing the solution to pass through the solid substance carrier. The instrument comprising a chip having a solid substance carrier fixed at the tip thereof is capable of moving a solution from one space to another space separated by the solid substance carrier, using pressurization and depressurization via a pipetter connected to the chip or a syringe, thereby causing the solution to pass through the solid substance carrier. The instrument comprising a spin column having a solid substance carrier fixed thereto that is adapted to be mounted on a centrifugal separator is capable of moving a solution from one space to another space separated by the solid substance carrier, using the centrifugal separator, thereby causing the solution to pass through the solid substance carrier.

Preferably, the solid substance carrier is a solid substance having a multitude of channels therein or a mesh-like solid substance composed of fibrous substances, which is capable of capturing cells from a biological sample including such cells. Also, preferably, the solid substance carrier is capable of binding nucleic acid using the action of a nucleic acid binding reagent including chaotropic substances or the like. For example, the solid substance carrier may be composed of substances including silicon oxide, such as glass fiber, glass particles, silica particles, silica wool, crushed materials thereof, or diatomaceous earth, and has pores with a maximum pore size of 2 to 20 µm. Also, materials composed of fiber or particles of polypropylene, polyester, nylon, or the like having pores with a maximum pore size of 2 to 20 µm may be combined therewith.

According to the present invention, release or isolation of nucleic acid from a biological sample can be performed readily and promptly.

### BRIEF DESCRIPTION OF THE DRAWINGS

Fig. 1 schematically shows a cross section of an isolation syringe.
Fig. 2 schematically shows a perspective view of an isolation syringe.
Fig. 3 schematically shows a cross section of a solid substance carrier fixed in an isolation syringe.
Fig. 4 schematically shows a cross section of an isolation syringe provided with a side opening.
Fig. 5 schematically shows a perspective view of an isolation syringe provided with a side opening.
Fig. 6 schematically shows a cross section of a spin column for isolation.
Fig. 7 schematically shows a perspective view of a spin column for isolation.

### DESCRIPTION OF A PREFERRED EMBODIMENT

In the following, the above and other novel features and effects of the present invention are described with reference to the drawings. It is noted, however, that the drawings are illustrative purposes only and are not intended to limit the present invention.

The present embodiment concerns a nucleic acid isolation method consisting of a first step of isolating cells from a biological sample, a second step of disrupting the cells to release nucleic acid, and a third step of binding the nucleic acid to a solid substance carrier and of washing and removing impurities so as to elute the nucleic acid from the solid substance carrier. In the present embodiment, the first, second, and third steps are performed using a single instrument holding a solid substance carrier, so that the operation is simplified and nucleic acid can be isolated in a short time. According to another embodiment, the first and second steps are performed using an instrument holding a predetermined solid substance carrier, so that the operation is simplified and nucleic acid can be released in a short time. According to yet another embodiment, the second and third steps are performed using an instrument holding a predetermined solid substance carrier, so that the operation is simplified and nucleic acid can be isolated in a short time.

Examples of the instrument fitted with a solid substance carrier used in the present embodiment include an instrument comprising a syringe having a solid substance carrier fixed therein, an instrument comprising a chip having a solid substance carrier fixed at the tip thereof, and an instrument comprising a spin column having a solid substance carrier fixed thereto that is adapted to be mounted on a centrifugal separator. The instrument comprising a syringe having a solid substance carrier fixed therein is capable of moving a solution from one space to another space separated by the solid substance carrier, using pressurization and depressurization via the syringe, thereby causing the solution to pass through the solid substance carrier. The instrument comprising a chip having a solid substance carrier fixed at the tip thereof is capable of moving a solution from one space to another space separated by the solid substance carrier, using pressurization and depressurization via a pipetter or a syringe connected to the chip, thereby causing the solution to pass through the solid substance carrier. The instrument comprising a spin column having a solid substance carrier fixed thereto that is adapted to be mounted on a centrifugal separator is capable of moving a solution from one space to another space separated by the solid substance carrier, using centrifugation, thereby causing the solution to pass through the solid substance carrier.

Preferably, the solid substance carrier is composed of substances including silicon oxide, such as glass fiber, glass particles, silica particles, silica wool, crushed materials thereof, or diatomaceous earth that are porous with a maximum pore size of 2 to 20 µm. These materials may be combined with other materials composed of fiber or particles of polypropylene, polyester, nylon, or the like, that are porous with a maximum pore size of 2 to 20 µm.

In a case where only the first and second steps are performed using the instrument holding a predetermined solid substance carrier, it is also possible to use a sole material composed of fiber or particles of polypropylene, polyester, nylon, or the like, having pores with a maximum pore size of 2 to 20 µm.

In the following, a basic procedure of nucleic acid isolation according to the present embodiment and samples and reagents used in the nucleic acid isolation are described.

The first step is a step of isolating cells from a biological sample. In the first step, using the instrument holding the solid substance carrier, a biological sample in a state of solution is caused to pass through the inside of the solid substance carrier from one space to another space separated by the solid substance carrier, thereby capturing cells included in the biological sample on the surface and in the inside of the solid substance carrier.

Examples of a biological sample including cells having nucleic acid include whole blood including leukocytes, body fluids including bacteria, urine, feces, cultured bacteria, and the like, as well as cultured cells and the like. For example, in the case of whole blood, a target is DNA or RNA of leukocytes included in such whole blood. In a case where the leukocytes are isolated from the whole blood, a preprocessing is preferably performed whereby erythrocytes are dissolved using an ammonium chloride solution or a salt solution, for example.

The second step is a step of disrupting the cells to release nucleic acid. In the second step, in the instrument used in the first step, a solution containing a chaotropic agent is caused to pass through the inside of the solid substance carrier from one space to another space separated by the solid substance carrier. As a result, the cells captured on the solid substance carrier are wetted with the solution and chemically dissolved. At the same time, the cells are caused to come into contact with the solid substance carrier, whereby disruption is physically promoted and nucleic acid is released.

Examples of a chaotropic agent include sodium iodide, potassium iodide, sodium thiocyanate, guanidine thiocyanate, and guanidine hydrochloride. A surface-active agent or proteolytic enzyme may also be added, in addition to the chaotropic agent.

The third step is a step of causing the nucleic acid to bind to the solid substance carrier and of washing and removing impurities so as to elute the nucleic acid from the solid substance carrier. In the third step, an organic solvent is added and mixed with the solution containing the chaotropic agent used in the second step. Then, in the instrument used in the second step, the mixture solution containing the nucleic acid in a released state, the chaotropic agent, and the organic solvent is caused to pass through the inside of the solid substance carrier from one space to another space separated by the solid substance carrier. This causes the nucleic acid to bind to the solid substance carrier. Examples of an organic solvent that can be used include one or more kinds of compounds with a carbon number of two to ten selected from the group consisting of aliphatic alcohol, aliphatic ether, aliphatic ester, and aliphatic ketone. Preferable examples of the aliphatic alcohol include ethanol, isopropanol, propanol, and butanol. Preferable examples of the aliphatic ether include ethylene glycol dimethyl ether, ethylene glycol diethyl ether, propione glycol dimethyl ether, propione glycol diethyl ether, diethylene glycol dimethyl ether, and diethylene glycol diethyl ether. Preferable examples of the aliphatic ester include propylene glycol monomethyl ether acetate, and ethyl lactate. Preferable examples of the aliphatic ketone include acetone, hydroxyacetone, and methyl ketone.

Next, in the instrument in which the nucleic acid has been bound, a washing reagent is caused to pass through the inside of the solid substance carrier from one space to another space separated by the solid substance carrier. This washes and removes impurities bound to the solid substance carrier. The washing reagent is selected such that it does not cause the nucleic acid bound to the solid carrier to be eluted and it allows for efficient removal of non-specifically bound substances. An example is a buffer solution of a low salt concentration containing an organic solvent such as ethanol. Optionally, a chaotropic agent or a surface-active agent may be added to the buffer solution of a low salt concentration containing an organic solvent such as ethanol.

Next, in the aforementioned instrument where the impurities have been washed and removed, an eluting solution is caused to pass through the inside of the solid substance carrier from one space to another space separated by the solid substance carrier. This elutes the nucleic acid bound to the solid substance carrier. In order to effectively elute the nucleic acid bound to the solid substance carrier and to avoid degradation of the properties of the nucleic acid, the elution reagent consists of pure water or a buffer solution of a low salt concentration that has been subjected to a nuclease removal or nuclease deactivation process.

### [Experiment 1]

In the present experiment, as an instrument holding a solid substance carrier, an isolation syringe holding the solid substance carrier was used to isolate RNA from whole blood as described below.

### <Instrument holding the solid substance carrier>

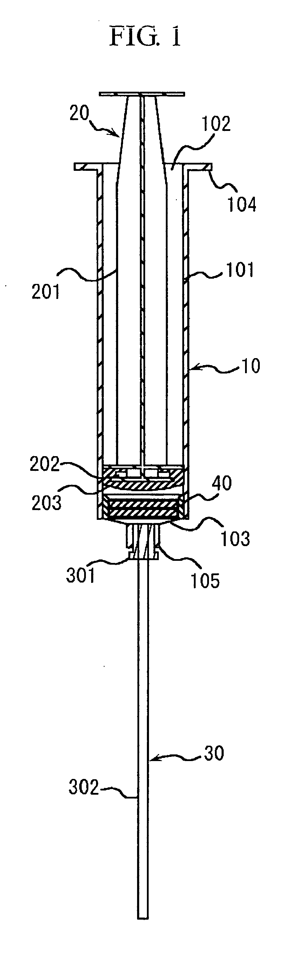

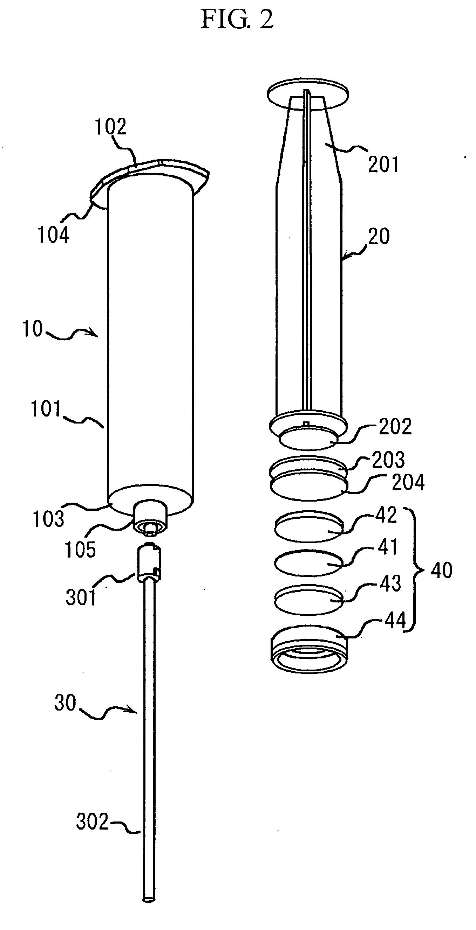

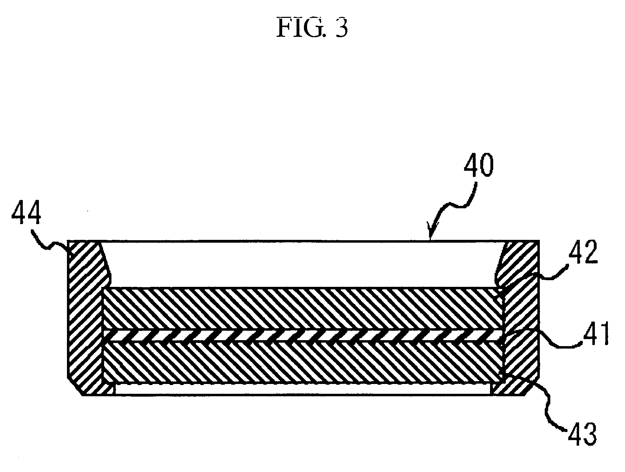

Fig. 1 schematically shows a cross section of the isolation syringe. Fig. 2 schematically shows a perspective view of the isolation syringe. Fig. 3 schematically shows a cross section of the solid substance carrier fixed to the isolation syringe. In the following, the isolation syringe is described with reference to Figs. 1 to 3.

The isolation syringe is an instrument comprising a Terumo 30 ml syringe (lock type) having a solid substance carrier fixed therein. The isolation syringe comprises a syringe body 10, a plunger 20, a nozzle 30, and a solid substance carrier unit 40. The nozzle side is referred to as a lower side, and the plunger side is referred to as an upper side.

The syringe body 10 has a cylinder portion 101, an opening 102 at the upper end, a bottom 103 at the lower end, a flange-shaped holding portion 104 disposed on the periphery of the opening 102, and a connection portion 105 for connecting a nozzle disposed at the bottom 103.

The plunger 20 includes a plunger body 201 and a seal piece 203. The seal piece 203, which is formed separately from the plunger body 201, is attached to an attachment portion 202 at the lower end of the plunger body 201. The seal piece 203 has a conical protrusion 204 at the lower end.

The nozzle 30 includes a connection portion 301 at the upper end and a cylindrical portion 302 extending downward therefrom. The connection portion 301 of the nozzle and the connection portion 105 of the isolation syringe body are connected using a screw.

The syringe body 10 and the plunger body 201 are formed of polypropylene, the seal piece 203 is formed of rubber, and the nozzle 30 is formed of stainless steel.

The solid substance carrier unit 40 comprises a disc-shaped solid substance carrier 41, two disc-shaped holding members 42 and 43 disposed at the upper side and the lower side of the solid substance carrier 41, and a cylindrical holder 44. The holding members 42 and 43 and the cylindrical holder 44 are formed of polypropylene.

The solid substance carrier 41 employs a material prepared by taking out sparse layers from a Whatman glass fiber filter (GMF 150) with layers disposed one on top of another, and then punching the resulting two-layered material into a disk using a punch-like cutter. The glass fiber filter (GMF 150) is capable of holding 2.0-µm particles and comprises a dense layer having a maximum pore size of about 2.0 µm and a sparse layer having a maximum pore size of about 10µm. In the present experiment, a solution is aspirated and dispensed a plurality of times using the instrument holding the solid substance carrier. Thus, liquid permeability (or liquid flow rate) is improved using only the sparse layer, and efficiency of capturing cells is improved with the use of a plurality of layers.

### <Biological sample>

As a biological sample, human whole blood including leukocytes was used and the isolation target was RNA of the leukocytes.

### <Reagents>

Red blood cell lysis reagent: 155 mM NH₄Cl, 10 mM KHCO₃, 0.1 mM EDTA
Chaotropic solution: 4M guanidine thiocyanate, 8 mM MES-KOH (pH5.5)
Organic solvent: 50% diethylene glycol dimethyl ether solution
Washing solution: 80% EtOH solution
Elution reagent: 10 mM Tris-HCl (pH 8.0), 0.1 mM EDTA

### <Containers>

Isolation container: a 50-ml centrifuge tube (made of polypropylene)
Container for an isolated product: a 5-ml centrifuge tube (made of polypropylene)

### <Nucleic acid isolation step>

The procedure of the nucleic acid isolation step is described in the following.
1. 2 ml of human whole blood is dispensed into the isolation container.
2. 10 ml of the red blood cell lysis reagent is added to the isolation container and mixed with the human whole blood.
3. The mixture is incubated on ice for five minutes.
4. The mixture is aspirated and dispensed using the isolation syringe, and the solution is discarded.
5. 2 ml of the chaotropic solution is aspirated and dispensed five times using the isolation syringe and discharged into the isolation container.
6. 2 ml of the organic solvent is added and mixed with the chaotropic solution discharged into the isolation container.
7. The mixture is aspirated and dispensed five times using the isolation syringe, and the solution is discarded.
8. 10 ml of the washing solution is aspirated and dispensed three times using the isolation syringe, and the solution is discarded.
9. 10 ml of the washing solution is aspirated and dispensed three times using the isolation syringe, and the solution is discarded.
10. 2 ml of the elution reagent is aspirated and dispensed ten times using the isolation syringe, and the solution is discharged into the container for an isolated product.

### <Isolation results>

Table 1 shows the concentration and purity of isolated nucleic acid according to the present experiment and the time required for the isolation operation. The nucleic acid concentration was slightly lower and the nucleic acid purity was equivalent to that resulting from the conventional method. The time required was substantially reduced in comparison with the conventional method. For the conventional method, a QIAGEN RNA isolation kit (QIAamp RNA Blood Mini Kit) was used and an isolation operation was performed in accordance with the relevant instruction manual. Specifically, leukocytes were isolated from blood using a centrifugal separator, and the leukocytes were dissolved using a homogenizer of a spin column type. Then, using an instrument of a spin column type holding a solid substance carrier, nucleic acid was bound, washed, and eluted. The concentration of the isolated nucleic acid was determined by measuring the absorbance at the maximum absorption wavelength of the nucleic acid, namely, 260 nm, using a spectrophotometer and then calculating according to 1A₂₆₀= 40 µg/ml. The nucleic acid purity was calculated as the ratio of the absorbance at 280 nm, which is the absorption wavelength of protein as an impurity, to the absorbance at 260 nm (A₂₆₀/A₂₈₀). In general, when the degree of nucleic acid isolation is high, the value of A₂₆₀/A₂₈₀ ranges from 1.7 to 1.9.

Conventionally, when isolating RNA from whole blood, it was necessary to use instruments such as a centrifugal separator, a homogenizer, and a nucleic acid isolation kit in the individual steps. Thus, the isolation operation was complicated and took a great deal of time. However, in the present experiment, all of the steps are performed using the same instrument, namely, the isolation syringe, whereby the isolation operation is simplified and the time required for the operation can be substantially reduced. The reduction of the time required for the operation also contributes to reducing the degradation of the properties of RNA by RNase during the isolation operation. Thus, although the concentration of the isolated nucleic acid becomes lower in comparison with the conventional method, the effects resulting from the simplification of the operation and the substantial reduction of the time required for the operation are significant.

### [Experiment 2]

In the present experiment, as the instrument holding a solid substance carrier, an isolation syringe having a side opening and holding a solid substance carrier was used to isolate RNA from whole blood as will be described in the following with reference particularly to the difference from Experiment 1.

### <Instrument holding the solid substance carrier>

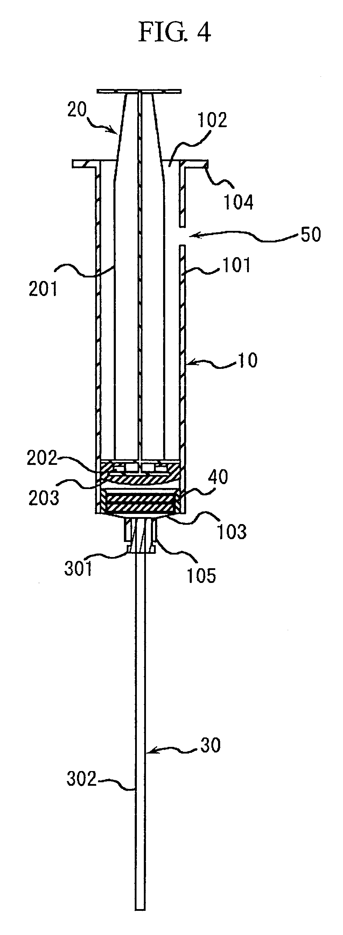

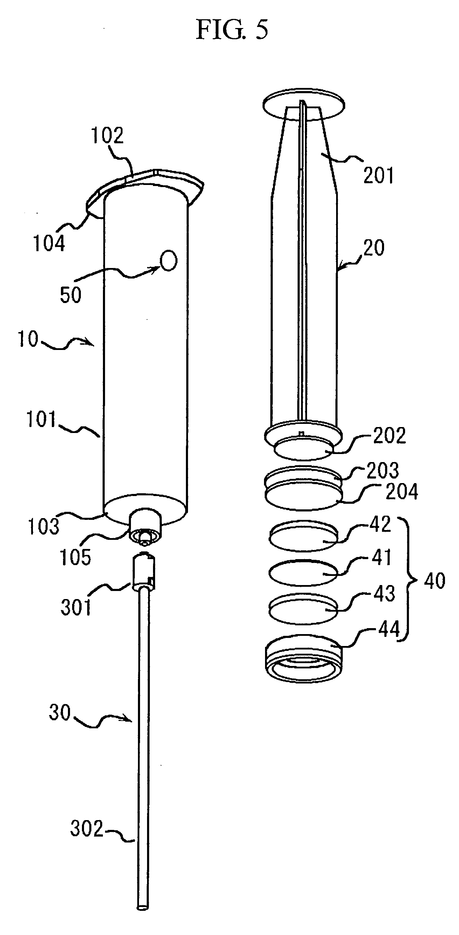

Fig. 4 schematically shows a cross section of the isolation syringe provided with a side opening. Fig. 5 schematically shows a perspective view of the isolation syringe provided with a side opening. In the following, the isolation syringe is described with reference to Figs. 4 and 5.

The isolation syringe provided with a side opening employs the isolation syringe of Experiment 1 where a side opening 50 is additionally provided at the side of the cylinder portion 101 of the syringe body 10 and where the solid substance carrier 41 is modified.

The side opening 50 of the isolation syringe is disposed above the uppermost position of the solid substance carrier unit 40 and below the lowermost position of the conical protrusion 204 of the seal piece 203 when the plunger 20 is lifted to the uppermost position. The diameter of the side opening 50 is of a size such that the tip of a pipet, for example, can be inserted therein. The isolation syringe provided with a side opening is capable of injecting a solution into the syringe body 10 from the side opening 50 using a pipet, for example, with the plunger 20 being lifted to the uppermost position. The injected solution can be passed through the solid substance carrier and discharged by lowering the plunger 20.

The solid substance carrier 41 employs a material prepared by punching a Whatman glass fiber filter (GMF150) to result in a disk-like shape via a punch-like cutter. The thus prepared material is disposed on the solid substance carrier unit 40 such that the sparse layer is positioned above the dense layer, of which the glass fiber filter (GMF150) consists. In the present experiment, the solution is discharged in one direction using the instrument holding the solid substance carrier. Thus, the efficiency of capturing cells is improved by using the dense layer, and the clogging in the solid substance carrier is prevented by disposing the sparse layer on top of the dense layer.

### <Biological sample>

The same as in Experiment 1

### <Reagents>

Red blood cell lysis reagent: 155 mM NH₄Cl, 10 mM KHCO₃, 0.1 mM EDTA
Chaotropic solution: 4M guanidine thiocyanate, 8 mM MES-KOH (pH5.5)
Organic solvent: 50% diethylene glycol dimethyl ether solution
Washing solution: 80% EtOH solution
Elution reagent: 10 mM Tris-HCl (pH 8.0), 0.1 mM EDTA

### <Containers>

The same as in Experiment 1

### <Nucleic acid isolation step>

The procedure of the nucleic acid isolation step is described in the following.
1. 2 ml of human whole blood is dispensed into the isolation container.
2. 10 ml of the red blood cell lysis reagent is added to the isolation container and mixed with the human whole blood.
3. The mixture is incubated on ice for five minutes.
4. The mixture is injected into the syringe from the side opening using a pipet with the plunger of the isolation syringe provided with a side opening lifted to the uppermost position.
5. The mixture inside the syringe is discharged by pressing down the plunger of the isolation syringe provided with a side opening.
6. The red blood cell lysis reagent is injected into the syringe from the side opening using the pipet with the plunger of the isolation syringe provided with a side opening lifted to the uppermost position.
7. The red blood cell lysis reagent inside the syringe is discharged by pressing down the plunger of the isolation syringe provided with a side opening.
8. 2 ml of the chaotropic solution is aspirated and dispensed five times using the isolation syringe and discharged into the isolation container.
9. 2 ml of the organic solvent is added and mixed with the chaotropic solution discharged into the isolation container.
10. The mixture is aspirated and dispensed five times using the isolation syringe, and the solution is discarded.
11. 10 ml of the washing solution is aspirated and dispensed three times using the isolation syringe, and the solution is discarded.
12. 10 ml of the washing solution is aspirated and dispensed three times using the isolation syringe, and the solution is discarded.
13. 2 ml of the elution reagent is aspirated and dispensed ten times using the isolation syringe, and the solution is discharged into the container for an isolated product. <Isolation results>

Table 1 shows the concentration and purity of isolated nucleic acid according to the present experiment and the time required for the isolation operation. The nucleic acid concentration increased in comparison with Experiment 1 and was almost the same as that of a conventional method. The nucleic acid purity and the time required were equivalent to that of Experiment 1.

In the present experiment, the mixture of the blood and the red blood cell lysis reagent was caused to pass through the solid substance carrier having a small pore size in one direction, namely, from the inside to the outside of the syringe by using the isolation syringe provided with a side opening in the step of isolating leukocytes from blood. This improved the isolation efficiency of leukocytes and increased the concentration of the isolated nucleic acid.

### [Experiment 3]

In the present experiment, as the instrument holding a solid substance carrier, a spin column for isolation holding a solid substance carrier was used to isolate RNA from whole blood as described in the following with reference particularly to the difference from Experiments 1 and 2.

### <Instrument holding the solid substance carrier>

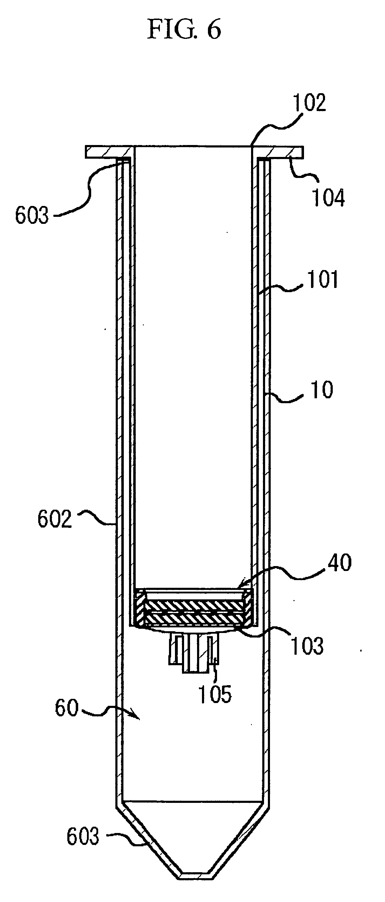

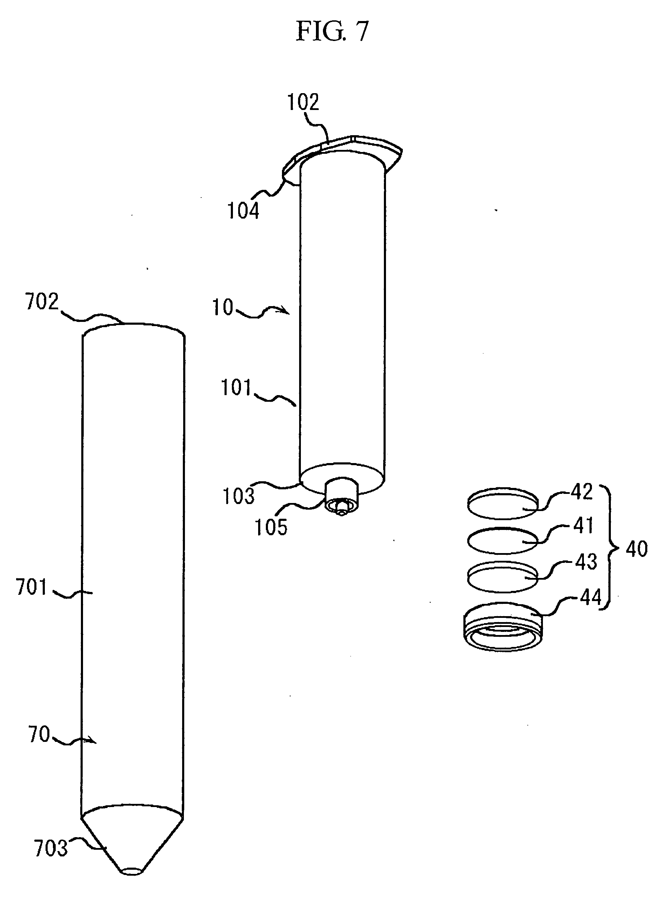

Fig. 6 schematically shows a cross section of the spin column for isolation. Fig. 7 schematically shows a perspective view of the spin column for isolation. In the following, the spin column for isolation is described with reference to Figs. 6 and 7.

The spin column for isolation comprises a column body 60, a solid substance carrier unit 40, and a solution-collecting container 70. The column body will be hereafter referred to as the lower side, and the solution-collecting container side will be hereafter referred to as the upper side.

The column body 60 has a cylinder portion 101, an opening 102 at the upper end, a bottom 103 at the lower end, and a flange-shaped holding portion 104 disposed on the periphery of the opening 102.

The solid substance carrier unit 40 is the same as that used in the isolation syringe.

The solution-collecting container 70 has a cylinder portion 701, an opening 702 at the upper end, and a bottom 703 at the lower end. The inner diameter of the cylinder portion 701 is larger than the outer diameter of the cylinder portion 101 of the column body. The outer diameter of the opening 702 at the upper end is smaller than the minor axis of the flange-shaped holding portion 104.

The column body 60 and the solution-collecting container 70 are formed of polypropylene.

A solution is injected into the column body 60 of the spin column for isolation and the column is subjected to centrifugation, whereby the solution inside the column body passes through the solid substance carrier and falls into the solution-collecting container via the opening of the column body 60. The solid substance carrier is the same as that used in Experiment 2.

### <Biological sample>

The same as in Experiment 1

### <Reagents>

The same as in Experiment 1

### <Containers>

The same as in Experiment 1

### <Centrifugal separator>

Centrifugal separator: Himac CR5B2 (Hitachi Koki)

### <Nucleic acid isolation step>

The procedure of the nucleic acid isolation step is described in the following.
1. 2 ml of human whole blood is dispensed into the isolation container.
2. 10 ml of the red blood cell lysis reagent is added to the isolation container and mixed with the human whole blood.
3. The mixture is incubated on ice for five minutes.
4. The mixture is injected into the column body from above the spin column for isolation.
5. The spin column for isolation is mounted on the centrifugal separator and subjected to centrifugation at 200 x g for one minute.
6. The solution in the solution-collecting container is discarded and 5 ml of the red blood cell lysis reagent is injected into the column body from above the spin column for isolation.
7. The spin column for isolation is mounted on the centrifugal separator and subjected to centrifugation at 300 × g for one minute.
8. The solution in the solution-collecting container is discarded and 2 ml of the chaotropic solution is injected into the column body from above the spin column for isolation.
9. The spin column for isolation is mounted on the centrifugal separator and subjected to centrifugation at 2000 × g for two minutes.
10. 2 ml of the organic solvent is added and mixed with the solution in the solution-collecting container, and the solution is injected into the column body from above the spin column for isolation.
11. The spin column for isolation is mounted on the centrifugal separator and subjected to centrifugation at 2000 × g for two minutes.
12. The solution in the solution-collecting container is discarded and 10 ml of the washing solution is injected into the column body from above the spin column for isolation.
13. The spin column for isolation is mounted on the centrifugal separator and subjected to centrifugation at 2000 × g for two minutes.
14. The solution in the solution-collecting container is discarded and 10 ml of the washing solution is injected into the column body from above the spin column for isolation.
15. The spin column for isolation is mounted on the centrifugal separator and subjected to centrifugation at 3000 × g for five minutes.
16. The solution in the solution-collecting container is discarded. A new solution-collecting container is disposed and 2 ml of the elution reagent is injected into the column body from above the spin column for isolation.
17. The spin column for isolation is mounted on the centrifugal separator and subjected to centrifugation at 2000 × g for two minutes.
18. The solution in the solution-collecting container is dispensed into the container for an isolated product.

### <Isolation results>

Table 1 shows the concentration and purity of isolated nucleic acid according to the present experiment and the time required for an isolation operation. The concentration and the purity of the isolated nucleic acid became almost the same as those of Experiment 2. The time required was longer in comparison with Experiment 1.

In the present experiment, by using the spin column for isolation, the solutions of all the steps are caused to pass through the solid substance carrier having a small pore size in one direction, namely, from the inside to the outside of the column body via the centrifugal separator. This elongates the time required in comparison with Experiment 1. However, the aspirating and dispensing operations of Experiment 1 are unnecessary, so that the effort necessary for the operation is reduced.

### [Experiment 4]

In the present experiment, as the instrument holding a solid substance carrier, an isolation/disruption syringe holding a solid substance carrier was used. RNA was isolated using an RNA isolation kit, after leukocytes were isolated from whole blood, and then disrupted to release the RNA as described in the following with reference particularly to the difference from Experiments 1, 2, and 3.

### <Instrument holding the solid substance carrier>

The isolation/disruption syringe holding a solid substance carrier employs the isolation syringe provided with a side opening of Experiment 2, where the solid substance carrier 41 comprises an aggregate of polypropylene fine fiber in a filter-like shape having a maximum pore size of about 5 µm in measurement. In the present experiment, leukocytes are only captured and disrupted using the solid substance carrier of the isolation/disruption syringe and nucleic acid is not bound. Thus, as the solid substance carrier, a filter comprising polypropylene fiber having capabilities of capturing and disrupting leukocytes is used, without the use of a glass fiber filter having a capability of binding with nucleic acid in the presence of a chaotropic agent or an organic solvent. In addition to polypropylene, polyester, nylon, and the like have adsorption to cells, so that efficiency of capturing leukocytes is high in comparison with a solid substance carrier such as a glass fiber filter.

### <Biological sample>

The same as in Experiment 1

### <Reagents>

Red blood cell lysis reagent: 155 mM NH₄Cl, 10 mM KHCO₃, 0.1 mM EDTA
Chaotropic solution: RLT Buffer (QIAGEN)
Organic solvent: 70% ethanol solution

### <Container>

Isolation container: a 50 ml centrifuge tube (made of polypropylene)

### <RNA isolation kit>

QIAamp RNA Blood Mini Kit (QIAGEN)

### <Nucleic acid isolation step>

The procedure of a nucleic acid isolation step is described in the following.
1. 2 ml of human whole blood is dispensed into the isolation container.
2. 10 ml of the red blood cell lysis reagent is added to the isolation container and mixed with the human whole blood.
3. The mixture is injected into the syringe from the side opening using a pipet with the plunger of the isolation/disruption syringe lifted to the uppermost position.
4. The mixture inside the syringe is discharged by pressing down the plunger of the isolation/disruption syringe.
5. The red blood cell lysis reagent is injected into the syringe from the side opening using the pipet with the plunger of the isolation/disruption syringe lifted to the uppermost position.
6. The red blood cell lysis reagent inside the syringe is discharged by pressing down the plunger of the isolation/disruption syringe.
7. 2 ml of the chaotropic solution is aspirated and dispensed five times using the isolation/disruption syringe and discharged into the isolation container.
8. 2 ml of the organic solvent is added and mixed with the chaotropic solution discharged into the isolation container.
9. The rest is performed in accordance with the instruction method of a commercial RNA isolation kit, where 2 ml of an elution reagent is used.

### <Isolation results>

Table 1 shows the concentration and purity of isolated nucleic acid according to the present experiment and the time required for an isolation operation. The concentration of the isolated nucleic acid was higher in comparison with a conventional method and the nucleic acid purity was the same. The time required was equivalent to that of Experiment 3.

In the present experiment, by using the isolation/disruption syringe, leukocytes are isolated from whole blood and the leukocytes are disrupted to release nucleic acid. Such an operation is a preprocessing operation of nucleic acid isolation conducted via the RNA isolation kit. The isolation of leukocytes using the isolation/disruption syringe has higher effects of leukocyte isolation than those of the method in Experiment 2, and even higher effects than those of the method of centrifugation described in the instruction manual of the RNA isolation kit. This is due to the fact that a polypropylene filter having higher leukocyte capturing efficiency than that of a glass fiber filter is used as the solid substance carrier of the isolation/disruption syringe. This increases the concentration of the isolated nucleic acid in comparison with a conventional method.

**Table 1**

| Nucleic acid isolation method | Exp. 1 | Exp. 2 | Exp. 3 | Exp. 4 | Conventional method |
|---|---|---|---|---|---|
| Nucleic acid concentration [µg/ml] | 2.08 | 2.50 | 2.35 | 2.84 | 2.75 |
| Nucleic acid purity [A260/A280] | 1.92 | 1.91 | 1.92 | 1.90 | 1.89 |
| Time required [minutes] | 15 | 15 | 25 | 25 | 50 |

## Claims

1. A method for isolating nucleic acid comprising:
causing a biological sample including cells to pass through a porous solid substance;
mixing cells captured in the porous solid substance with a reagent for disrupting cells, and causing a resultant first mixture to pass through the porous solid substance; and
mixing the first mixture containing nucleic acid released from the cells with a binding reagent for causing the nucleic acid to bind to the porous solid substance, and causing a resultant second mixture to pass through the porous solid substance.

2. The method for isolating nucleic acid according to claim 1, wherein the biological sample including the cells is whole blood, the cells are leukocytes, and the nucleic acid is RNA.

3. A method for isolating nucleic acid comprising:
mixing cells with a reagent for disrupting cells, and causing a resultant first mixture to pass through a porous solid substance; and
mixing the first mixture containing nucleic acid released from the cells with a binding reagent for causing the nucleic acid to bind to the porous solid substance, and causing a resultant second mixture to pass through the porous solid substance.

4. The method for isolating nucleic acid according to claim 3, wherein the cells are leukocytes, and the nucleic acid is RNA.

5. The method for isolating nucleic acid according to any of claims 1 to 4, involving the use of a pipe in which the porous solid substance is housed such that the inside of the pipe is divided thereby, wherein the biological sample, the first mixture, and the second mixture are aspirated and dispensed from one end of the pipe via a change of pressure.

6. The method for isolating nucleic acid according to any of claims 1 to 4, involving the use of a pipe in which the porous solid substance is housed such that the inside of the pipe is divided thereby, wherein the biological sample, the first mixture, and the second mixture are caused to pass from one side to another side of the pipe through such division via centrifugal force.

7. A method for isolating nucleic acid comprising:
causing a biological sample including cells to pass through a porous solid substance; and
mixing cells captured in the porous solid substance with a reagent for disrupting cells, and causing a resultant mixture to pass through the porous solid substance.

8. The method for isolating nucleic acid according to claim 7, wherein the biological sample including cells is whole blood.

9. The method for isolating nucleic acid according to claim 7 or 8, involving the use of a pipe in which the porous solid substance is housed such that the inside of the pipe is divided thereby, wherein the biological sample and the mixture are aspirated and dispensed from one end of the pipe via a change of pressure.

10. The method for isolating nucleic acid according to claim 7 or 8, involving the use of a pipe in which the porous solid substance is housed such that the inside of the pipe is divided thereby, wherein the biological sample and the mixture are caused to pass from one side to another side of the pipe through such division via centrifugal force.

11. The method for isolating nucleic acid according to any preceding claim, wherein the porous solid substance includes glass fibre, a glass fibre filter, glass particles, silica particles, or silica wool.

12. The method for isolating nucleic acid according to any preceding claim, wherein the porous solid substance includes fibre or particles of polypropylene, fibre or particles of polyester, or fibre or particles of nylon.

13. The method for isolating nucleic acid according to any preceding claim, wherein the maximum pore size of the porous solid substance ranges from 2 to 20 *µ*m.
